(19) Europäisches Patentamt
European Patent Office
Office européen des brevets

(11) Publication number: **0 125 093**
**A1**

(12) # EUROPEAN PATENT APPLICATION

(21) Application number: 84302929.9

(22) Date of filing: 01.05.84

(51) Int. Cl.[3]: **C 07 D 295/20**
C 07 D 243/04, A 61 K 31/50
A 61 K 31/55

(30) Priority: 09.05.83 GB 8312660

(43) Date of publication of application:
14.11.84 Bulletin 84/46

(84) Designated Contracting States:
AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Eakin, Murdoch Allan
2 Cockhall Lane Langley
Macclesfield Cheshire(GB)

(72) Inventor: Gunn, Donald Murray
16 Merlin Park Dollar
Clackmannan Scotland FK14 7BZ(GB)

(72) Inventor: Pemberton, Denis
9 Eskdale Avenue
Bramhall Stockport Cheshire(GB)

(74) Representative: Atkinson, John David et al,
Imperial Chemical Industries PLC Legal Department :
Patents PO Box 6 Bessemer Road
Welwyn garden City Herts, AL7 1HD(GB)

(54) **Diaz(ep)ine bis(N-amidinoamidine) derivatives.**

(57) A diaz(ep)ine bis(N-amidinoamidine) derivative of the formula:-

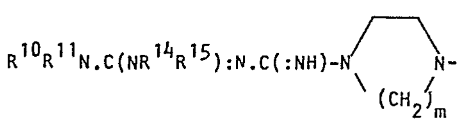

I

C(:NH)N:C(NR[16]R[17])-NR[12]R[13]

or a tautomeric form thereof, wherein $R^{10}$, $R^{11}$, $R^{12}$, $R^{13}$, $R^{14}$, $R^{15}$, $R^{16}$ and $R^{17}$, which may be the same or different, are each hydrogen, a 1-16C alkyl radical, a 3-18C cycloalkyl or cycloalkylalkyl radical, or an optionally-substituted phenyl (1-4C alkyl) radical, or $R^{10}$, $R^{11}$ and the nitrogen atom to which they are attached, or $R^{12}$ and $R^{13}$ and the nitrogen atom to which they are attached, or $R^{14}$ and $R^{15}$ and the nitrogen atom to which they are attached, or $R^{16}$ and $R^{17}$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, or $R^{10}$ and $R^{14}$ together with the two nitrogen atoms to which they are attached and the intervening carbon atom, or $R^{12}$ and $R^{16}$ together with the two nitrogen atoms to which they are attached and the intervening carbon atom, each forms a 1,3-diazaheterocyclic radical of 5 to 7 atoms, which heterocyclic radicals may be the same or different; and m is 2 or 3; provided that $R^{10}$, $R^{11}$, $R^{12}$, $R^{13}$, $R^{14}$, $R^{15}$, $R^{16}$ and $R^{17}$ together contain 16 to 28 carbon atoms, and the acid addition salts thereof, processes for their manufacture and antibacterial and antifungal compositions and methods using said compounds.

EP 0 125 093 A1

0125093

SUBSTANTIVE SPECIFICATION

DIAZ(EP)INE BIS(N-AMIDINOAMIDINE) DERIVATIVES

This invention relates to novel piperazine-1,4-bis(N-amidinoamidine)derivatives, which possess antibacterial properties.

United Kingdom Patent Number 855,017 discloses biguanide derivtives of the formula:-

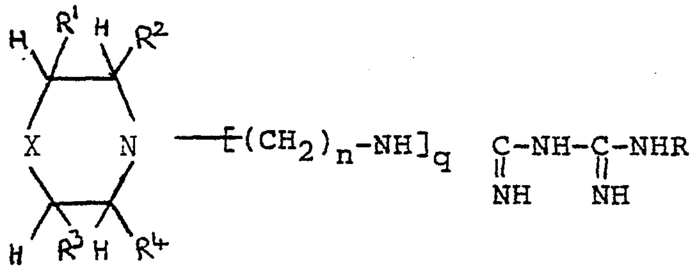

wherein R is aryl optionally substituted by one or more or any combination of alkyl, aryl, aralkyl, alkaryl, alkoxy, nitro and halogen, X is oxygen, -N(lower alkyl or

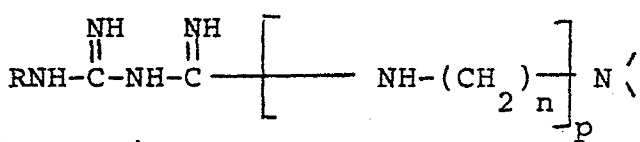

$R^1$, $R^2$, $R^3$ and $R^4$ are hydrogen or lower alkyl, n is an integer of from 2 to 6 and the polymethylene chain may be interrupted by an oxygen atom, and p and q, which may be the same or different, are each 0 or 1. These compounds are said to show bactericidal activity.

A paper by J W James et al in Journal of Medicinal Chemistry, 1968, volume 11, pages 942-945, gives further details of the bactericidal activity of some of the above compounds, but in addition discloses three alkyl analogues of the above compounds of the formula:-

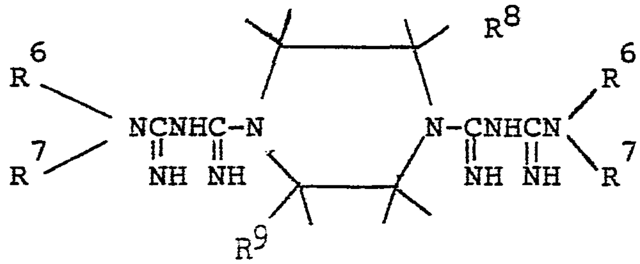

wherein $R^6$ and $R^7$ are both methyl, and $R^8$ and $R^9$ are both hydrogen, both methyl, or one is hydrogen and one is methyl. These alkyl analogues, however, are stated to be inactive antibacterially - "All of the compounds....were screened initially for antibacterial activity by the conventional <u>in vitro</u> "zone of inhibition" method against a variety of bacteria. None of the......$N^5$-alkyl-substituted biguanides showed any activity at all".

The present invention is based upon the discovery that certain bis(N-amidinoamidines), of the type generally known, but possessing two terminal alkyl substituents on each of the amidinoamidine residues, unexpectedly possess high antibacterial activity.

Thus, according to the invention, there is provided a diaz(ep)ine bis(N-amidinoamidine) derivative of the formula:-

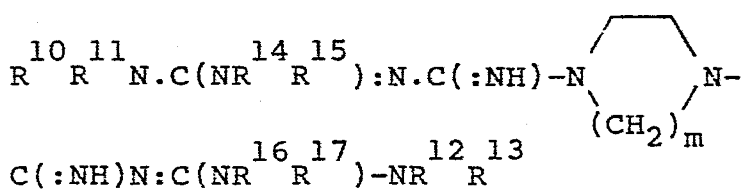

I

or a tautomeric form thereof, wherein $R^{10}$, $R^{11}$, $R^{12}$, $R^{13}$ $R^{14}$, $R^{15}$, $R^{16}$ and $R^{17}$, which may be the same or different, are each hydrogen, a 1-16C alkyl radical, a 3-18C cycloalkyl or cycloalkyl-alkyl radical, or an optionally-substituted phenyl (1-4C alkyl) radical, or $R^{10}$, $R^{11}$ and the nitrogen atom to which they are attached, or $R^{12}$ and $R^{13}$ and the

nitrogen atom to which they are attached, or $R^{14}$ and $R^{15}$ and the nitrogen atom to which they are attached, or $R^{16}$ and $R^{17}$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, or $R^{10}$ and $R^{14}$ together with the two nitrogen atoms to which they are attached and the intervening carbon atom, or $R^{12}$ and $R^{16}$ together with the two nitrogen atoms to which they are attached and the intervening carbon atom, each forms a 1,3-diaza-heterocyclic radical of 5 to 7 atoms, which heterocyclic radicals may be the same or different; and m is 2 or 3; provided that $R^{10}$, $R^{11}$, $R^{12}$, $R^{13}$, $R^{14}$, $R^{15}$, $R^{16}$ and $R^{17}$ together contain 16 to 28 carbon atoms, and the acid addition salts thereof.

Suitable values for $R^{10}$, $R^{11}$, $R^{12}$, $R^{13}$, $R^{14}$, $R^{15}$, $R^{16}$ or $R^{17}$, when they are alkyl radicals are, for example, methyl, ethyl, propyl, isopropyl, butyl, isobutyl, s-butyl, tert-butyl, pentyl, neopentyl, hexyl, heptyl, octyl, 2-ethylhexyl, decyl, dodecyl, tetradecyl and hexadecyl radicals.

Suitable values for $R^{10}$, $R^{11}$, $R^{12}$, $R^{13}$, $R^{14}$, $R^{15}$, $R^{16}$ or $R^{17}$, when they are cycloalkyl or cycloalkyl-alkyl radicals are, for example, cyclopentyl, cyclohexyl, cyclo-octyl, cyclododecyl, cyclohexadecyl, cyclopentylmethyl, cyclohexylmethyl and cyclo-octylmethyl radicals.

Suitable values for $R^{10}$, $R^{11}$, $R^{12}$ $R^{13}$, $R^{14}$, $R^{15}$, $R^{16}$ or $R^{17}$, when they are optionally-substituted phenylalkyl radicals are, for example, optionally-substituted-benzyl, -phenethyl, -1-phenylethyl, -3-phenylpropyl, -1-phenylpropyl or -1-methyl-1-phenylethyl radicals.

Suitable optional substituents in the phenyl rings of $R^{10}$, $R^{11}$, $R^{12}$, $R^{13}$, $R^{14}$, $R^{15}$, $R^{16}$ or $R^{17}$, when they are optionally-substituted phenylalkyl radicals are, for example, halogen atoms, for example chlorine, bromine, iodine or fluorine atoms, amino, carbamoyl, cyano, hydroxy, nitro and trifluoromethyl radicals, 1-6C alkyl, alkoxy, alkanoyl, alkylamino and alkanoylamino radicals and 2-6C alkoxycarbonyl and dialkylamino radicals. Suitable such radicals are, for example, methyl, ethyl, propyl, butyl, pentyl, hexyl, methoxy, ethoxy, propoxy, acetamido, propionamido, butyramido, methylamino, ethylamino, propylamino, acetyl, propionyl, butyryl, methoxycarbonyl, ethoxycarbonyl, dimethylamino and diethylamino radicals. Up to five such substituents may be present, but mono- and di- substituted phenyl rings are preferred, and especially mono-substituted rings.

Thus, suitable values for $R^{10}$, $R^{11}$, $R^{12}$, $R^{13}$, $R^{14}$, $R^{15}$, $R^{16}$ or $R^{17}$ when they are substituted phenylalkyl radicals are, for example, 2-, 3- and 4-chlorobenzyl, 2-, 3- and 4-bromobenzyl, 2-, 3- and 4-fluorobenzyl, 2,3-, 2,4-, 2,5-, 2-6-, 3,4- and 3,5-dichlorobenzyl, 2-chloro-4-fluorobenzyl, 2-, 3- and 4-methylbenzyl, 2,3-, 2,4-, 2,5-, 2,6-, 3,4- and 3,5-dimethylbenzyl, 2-, 3- and 4-methoxybenzyl, 2-, 3- and 4- acetylbenzyl, 2-, 3-and 4-methylaminobenzyl, 2-, 3- and 4-acetamido-benzyl, 2-, 3- and 4-methoxycarbonyl-benzyl, 2-, 3- and 4-dimethylaminobenzyl, 2-, 3- and 4-nitrobenzyl, 2-, 3-and 4-chloro-α-methylbenzyl, 2-, 3- and 4-chlorophenethyl and 1-(4-chlorophenyl)-1-methylethyl radicals.

A suitable value for the 1,3-diaza-heterocyclic radical of 5 to 7 atoms is, for example, a 2-imidazolyl, 2-pyrimidinyl or 1,3-diazepin-2-yl radical.

The acid-addition salts of the invention may be derived from an inorganic or organic acid. In most circumstances it is preferable that the salts be derived from an acid which affords an anion which is suitable for human usage, for example a pharmaceutically-acceptable anion. Examples of such acids are hydrochloric, hydrobromic, phosphoric, sulphuric, acetic, D-gluconic, 2-pyrrolidone-5-carboxylic, methanesulphonic, carbonic, lactic, glutamic, octanoic, palmitic, pivalic, tartaric, ethanesulphonic, pyroglutamic and sulphamic acids. Other suitable anions are anions of acids which themselves possess pharmaceutical, preferably antibacterial, activity. A preferred such anion is that derived from the antibacterial sulphonamide, sulphadiazine.

A preferred value for m is 2.

Preferred compounds are those in which each of $R^{10}$, $R^{11}$, $R^{12}$ and $R^{13}$ is an alkyl radical, and $R^{14}$, $R^{15}$, $R^{16}$ and $R^{17}$ are hydrogen, and particularly preferred are such compounds wherein $R^{10}$, $R^{11}$, $R^{12}$ and $R^{13}$ are alkyl radicals which together contain 18-24, particularly 20 carbon atoms.

Particular preferred compounds of the invention are piperazine-1,4-di[N-(N,N-dipentylamidino)carboxamidine], piperazine-1,4-di[N-(N,N-di-isopentylamidino)carboxamidine], piperazine-1,4-di[N-(N-butyl- N-pentylamidino)carboxamidine], 4-[N-(N,N-dipentylamidino]piperazine-1- [N-(N,N-di-isopentylamidino)carboxamidine], piperazine-1,4-di-(N-[N-butyl-N-(1,4-dimethylpentyl)amidino]-carboxamidine), piperazine-1,4- di[N-($N^1N^1$-di-isopentyl- $N^2$-methylamidino)carboxamidine] and 1,4-bis[N-(2,3,4,5,6,7-hexahydro-1,3-di-isopentyl-1*H*-1,3-diazepin-2-ylidene)amino]piperazine, and their dihydrochlorides, dimesylates and dilactates.

The compounds of the invention of the formula I may be manufactured by methods known generally for the synthesis of analogous compounds. Thus, according to a further feature of the invention there is provided a process for the manufacture of a compound of the formula I wherein $R^{14}$, $R^{15}$, $R^{16}$ and $R^{17}$ are each hydrogen, which comprises reacting piperazine-1,4 di(N-cyanoamidine) or 2,3,4,5,6,7-hexahydro-1*H*-1,4-diazepine-1,4-di(N-cyanoamidine) either with an amine $R^{10}R^{11}NH$,if the required product is symmetrical, or with an amine $R^{10}R^{11}NH$ and an amine $R^{12}R^{13}NH$, either simultaneously or sequentially, if the required product is unsymmetrical, the amine or amines being in the form of an acid-addition salt thereof, wherein $R^{10}$, $R^{11}$, $R^{12}$ and $R^{13}$ have the meanings stated above, at a temperature of 100 to 170°C.

A suitable amine salt is, for example, the hydrochloride. The reactants are heated together until the reaction is complete. The reaction proceeds fastest at higher temperatures, but if thermal stability is a problem, the reaction is carried out at lower temperature over a longer period. The reactants are most conveniently melted together in the absence of a solvent, but if desired an inert solent such as 2-methoxyethanol, 2-ethoxyethanol, nitrobenzene, sulpholane, n-butanol, ethylene glycol dimethyl ether or water, or a mixture of such solvents, may be used.

According to a further feature of the invention there is provided a process for the manufacture of a compound of the formula I which comprises reacting piperazine or 2,3,4,5,6,7-hexahydro-1*H*-1,4-diazepine in the form of an acid-addition salt thereof, either with a cyanoguanidine of the formula $R^{10}R^{11}N.C(NR^{14}R^{15}):N.CN$, if the required product is symmetrical, or with a cyanoguanidine $R^{10}R^{11}N.C(NR^{14}R^{15}):N.CN$ and a cyanoguanidine $R^{12}R^{13}N.C(NR^{16}R^{17}):N.CN$, either simultaneously or sequentially, if the required product is unsymmetrical, wherein $R^{10}$, $R^{11}$, $R^{12}$, $R^{13}$, $R^{14}$, $R^{15}$, $R^{16}$ and $R^{17}$ have the meanings stated above, at a temperature of 100 to 170°C.

A suitable amine salt is, for example, the hydrochloride. The reactants are heated together until the reaction is complete. The reaction proceeds fastest at higher temperatures, but if thermal stability is a problem, the reaction is carried out at lower temperature over a longer period. The reactants are most conveniently melted together in the absence of a solvent, but if desired an inert solvent such as 2-methoxyethanol, 2-ethoxyethanol, nitrobenzene, sulpholane, n-butanol, ethylene glycol dimethyl ether or water, or a mixture of such solvents, may be used.

Piperazine-1,4-di(N-cyanoamidine), used as starting material in the first of the above processes, may be obtained by reacting a piperazine salt with sodium dicyanamide in conventional manner. The corresponding diazepine derivative is obtained in similar manner.

The cyanoguanidines of the formulae $R^{10}R^{11}N.C(NR^{14}R^{15}):N.CN$ and $R^{12}R^{13}N.C.(NR^{16}R^{17}):N.CN$ may be obtained by reacting sodium dicyanamide with an appropriate amine $R^{10}R^{11}NH$ or $R^{12}R^{13}NH$ in an inert solvent, in conventional manner.

The acid-addition salts of the invention are obtained by conventional means.

The antibacterial activity of the compounds of the invention has been measured by the well-known minimum inhibitory concentration (MIC) test. Neat or diluted broth cultures of eight Gram positive organisms (Streptococcus pyogenes, S. faecalis, 3 strains of Staphylcoccus aureus, Listeria monocytogenes, Streptococcus mutans, S. sanguis), Candida albicans and fourteen Gram negative organisms (4 strains of Escherichia coli, Salmonella dublin, Klebsiella aerogenes, K. pneumoniae, E.cloacae, Serratia marcescens, Proteus vulgaris, P. mirabilis and 3 strains of Pseudomonas aeruginosa) were inoculated by means of an automatic microtitre inoculator on the surface of nutrient agar plates containing two-fold or five-fold dilutions of a test compound. After incubation overnight at 37°C., the geometric mean MIC's for the eight Gram positive organisms plus Candida, and 14 Gram negative organisms are then calculated for each test compound.

Depending upon its precise chemical structure a compound of the invention has a geometric mean MIC within the range 1-12 µg./ml. in agar against the 8 Gram positive organisms and Candida, and 20-250 µg./ml. in agar against the 14 Gram negative organisms.

The preferred compounds of the invention have an acute $LD_{50}$ within the accepted limits for compounds used topically, are of low irritancy in the Draize test on intact rabbit skin, are negative in the Ames test for mutagenicity, and are non-sensitizing in the Magnusson and Kligman contact sensitivity test in guinea-pigs.

Because of their antibacterial and antifungal properties, the compounds of the invention are useful for many purposes, for example:-

a) in medical and veterinary practice for the disinfection of wounds, membranes and/or skin tissue;

b) for the sterilisation of surgical instruments and other medical apparatus and equipment, for example respirators, ventilators, incubators, humidifiers, etc.;
c) for incorporation in toothpastes and mouthwashes for inhibiting the formation of dental plaque, and gingivitis;
d) for the disinfection of hard surfaces, for example plant and equipment used in the food and drink industries, and floors and walls in the home, factories and hospitals;
e) for the disinfection of textiles, for example blankets, overalls, bed-linen, etc.;
f) for the control of microbiological slime in the plup and papers industries;
g) for the control of micro-organisms in swimming pools, cooling water, pasteuriser water, aqueous oil emulsions such as metal working fluids, and other circulating water systems; and
h) as plant bactericides and fungicides.

A drawback to the use of chlorhexidine is that if it contacts white textiles, which are subsequently laundered with hypochlorite bleach as in hospital laundries, dark stains develop. It is an advantage of the preferred compounds of this invention that such staining is non-existent, or minimal.

Compounds of the invention also possess useful antifungal activity, for example, against Candida albicans or Trichophyton mentagrophytes, and algicidal and anti-yeast activity.

According to a further feature of the invention there are provided antibacterial or antifungal compositions comprising a compound of the formula I wherein $R^{10}$, $R^{11}$, $R^{12}$ and $R^{13}$ have the meanings stated above, or an acid-addition

salt thereof, and an inert diluent or carrier therefor.

The antibacterial or antifungal compositions of the invention are prepared by conventional means using conventional excipients. They include, for example, aqueous solutions, for example concentrated aqueous solutions, sterile, ready-to-use aqueous solutions, aqueous dispersions, and emulsions, for example oil-in-water emulsions, for example aqueous gels, creams, ointments and pastes. Suitable excipients include, for example, wetting agents, dispersing agents, emulsifying agents, gelling agents and thickening agents.

According to a further feature of the invention, there is provided a contraceptive method which comprises applying to sperm, or the locus of sperm, a spermicidal, sperm-immobilising or mucospissic amount of a compound of the invention of the formula I.

In one aspect of this method, the compound of the formula I, when applied to vaginal mucus at a suitable concentration, very rapidly increases its viscosity, to the extent that it becomes essentially impenetrable to sperm, and forms a physical barrier to conception in the same way as a rubber sheath or a diaphragm cap.

Besides increasing the viscosity of vaginal mucus, when the mucus comes into contact with a compound of the formula I, other changes occur in its intrinsic properties, such as its morphology, rheology and water uptake and visco-elastic properties, which can also effect is penetrability to sperm. The compounds also possess spermicidal or sperm-immobilising properties.

*In vitro*, the compounds of the formula I exert a useful contraceptive effect at concentrations down to about $10^{-3}$ to $10^{-4}$%, and a suitable amount to be applied to the human vagina for contraceptive purposes is from 1.0 g. to $10^{-4}$ g.

The compound of the formula I may be applied to the vagina in conventional manner, for example as a pessary, cream, liquid douche, gel, aerosol foam or impregnated tampon, or in a controlled delivery device of the compound in a polymer matrix.

According to a further feature of the invention there is provided a compound of the formula I or a composition thereof, for use as a contraceptive.

The invention is illustrated but not limited by the following Examples in which the temperatures are expressed in degrees Celsius.

Example 1

3-Cyano-1,1-dipentylguanidine (1.2 g.), piperazine dimesylate (0.56 g.) and butanol (3 ml.) were stirred together in an oil bath at 140° for 2.5 hours. The reaction mixture was allowed to cool and the butanol was evaporated under reduced pressure. Acetone (100 ml.) was added to the colourless residue, the mixture was heated briefly under reflux and then filtered hot, and the solid product was washed with warm petroleum ether (b.p. 60-80°) to give piperazine-1,4-di[N-(N,N-dipentylamidino)carboxamidine]dimesylate, m.p. 223-224°.

3-Cyano-1,1-dipentylguanidine used in the above example was prepared as follows:

Dipentylamine hydrochloride (19.45g.) and sodium dicyanamide (8.9g.) were heated together in butanol (100ml.), under reflux for 4 hours. The reaction mixture was allowed to cool, was filtered to remove solid material, and the filtrate was evaporated to dryness under reduced pressure to give an oil which could be crystallised from petroleum ether (b.p. 60-80°) at low temperature. The solid was recrystallised from petroleum ether (b.p. 60-80°) to give 3-cyano-1,1-dipentylguanidine, m.p. 54°.

Example 2

3-Cyano-1,1-di-isopentylguanidine (9.5 g.), piperazine bis-trifluoroacetate (5.3 g.) and butanol (6 ml.) were heated together in an oil bath at 130° for 3.5 hours. The reaction mixture was allowed to cool, acetone (50 ml.) was added, and the solid produced was filtered off and washed with acetone to give piperazine-1,4-di[N-(N,N-di-isopentylamidino)carboxamidine] trifluoroacetate, m.p. 221°.

Examples 3-15

The process described in Example 1 was repeated, using the appropriate cyanoguanidine in place of 3-cyano-1,1-dipentylguanidine, and piperazine dihydrochloride, to give the following compounds of the formula I ($R^{14}=R^{15}=R^{16}=R^{17}$=hydrogen, m=2) which were purified either by crystallisation of the dihydrochloride salts or by reverse phase medium pressure liquid chromatography (MPLC):-

| | | | REACTION | | | CRYSTALLISATION (OR MPLC PURIFICATION) PURIFICATION) | |
|---|---|---|---|---|---|---|---|
| No. | $R^{10}(=R^{12})$ | $R^{11}(=R^{13})$ | SOLVENT | TEMP.° | TIME (hours) | m.p.° | SOLVENT |
| 3 | i-pentyl | i-butyl | BuOH | 116-118 | 6 | 283-5 | Water |
| 4 | pentyl | i-butyl | PrOH | 96-98 | 27 | 243-4 | Water |
| 5 | pentyl | butyl | BuOH | 116-118 | 4.5 | 235-6 | Water |
| 6 | i-pentyl | i-pentyl | BuOH | 116-118 | 3.5 | 244-5 | i-PrOH |
| 7 | hexyl | ethyl | PrOH | 96-98 | 24 | 240-1 | (55% MeOH 45% $H_2O$) |
| 8 | hexyl | i-butyl | PrOH | 96-98 | 23 | 240-2 | Water |
| 9 | hexyl | butyl | PrOH | 96-98 | 16 | 220-2 | Water |
| 10 | 1,4-dimethyl-pentyl | butyl | PrOH | 96-98 | 24 | 217-20 | (55% MeOH/45% $H_2O$) |
| 11 | 1,5-dimethyl-hexyl | ethyl | PrOH | 96-98 | 16 | 239-41 | Water |
| 12 | 2-ethyl-hexyl | methyl | BuOH | 116-118 | 4 | 269-70 | (55% MeOH/45% $H_2O$) |
| 13 | 2-ethyl-hexyl | propyl | PrOH | 96-98 | 16 | 250-1 | Water |
| 14 | i-butyl | cyclohexyl | BuOH | 116-118 | 3 | 252-4 | i-PrOH/BuOH |
| 15 | benzyl | cyclohexyl | sulpholane | 160 | 3 | 241-2 | (55% MeOH/45% $H_2O$ |

The cyanoguanidine starting materials used in the manufacture of the above compounds were manufactured by the process described generally in the second part of Example 1.

The N-butyl-1,4-dimethylpentylamine, which is required as starting material for the cyanoguanidine starting material for Example 10, was prepared as follows:-

To a stirred solution of butyryl chloride (12.5 ml) in anhydrous toluene colled to -70°, 1,4-dimethylpentylamine (30 g.) was added dropwise. The mixture was allowed to warm to room temperature, then stirred for 2 hours, hexane (100 ml.) was added, and the mixture was poured onto ice plus an excess of hydrochloric acid. The organic phase was separated, washed with water (3 x 250 ml.) and 5% sodium bicarbonate solution (3 x 250 ml.), then dried over sodium sulphate. Evaporation of the solvents under reduced pressure, followed by distillation of the residue gave N-(1,4-dimethylpentyl)-butyramide, b.p. 102-5° at 0.5 mm. mercury pressure, (67Pa).

N-(1,4-Dimethylpentyl)butyramide (18.7 g.) was dissolved in anhydrous diethyl ether (57 ml.) and the solution was added dropwise over 1.5 hours to a stirred slurry of lithium aluminium hydride (5.7 g.) in anhydrous diethyl ether (60 ml.) under an atmosphere of argon. The mixture was stirred for 16 hours, then a saturated aqueous solution of sodium sulphate was added dropwise until a granular precipitate formed. The mixture was filtered and the filtrate was evaporated under reduced pressure to give an oil. The oil was acidified with dilute hydrochloric acid and the mixture was evaporated to dryness under reduced pressure. Crystallisation of the residue from toluene/petroleum ether (b.p. 60-80°), 1:1, gave N-butyl-1,4-dimethylpentylamine hydrochloride, m.p. 85-98°. The crude produce was homogeneous by thin layer

chromatography (t.l.c.), and was used without further purification.

The 2-ethyl-N-propylhexylamine used as starting material for the cyanoguanidine starting material for compound 13 was prepared as follows:-

Propionaldehyde (14.52 ml.) and 2-ethylhexylamine (32.9 ml.) were stirred together at 25° for 45 minutes. Potassium hydroxide flakes (11.2 g.) were added and the mixture was stirred at 25°C. for 15 minutes. The top layer was separated and diluted with absolute ethanol (100 ml.) and the mixture was cooled and stirred at 15°. Sodium borohydride (8 g.) was added portionwise below 35° and the final mixture was stirred at 25° for 14 hours. Water (200 ml.) was added, the product was extracted into methylene dichloride (4 x 50 ml.) and the combined extracts were dried over magnesium sulphate and then evaporated to half volume. Methanol (100 ml.) was added, the solution was stirred at 15°, and concentrated aqueous hydrochloric acid (20 ml.) was added dropwise with stirring below 30°. The resulting solution was evaporated to give a gum, which was crystallised from ethyl acetate to give 2-ethyl-N-propylhexylamine hydrochloride, m.p. 126-127.5°.

Example 16

A mixture of piperazine dihydrochloride monohydrate (21.24 g.), 3-cyano-1,1-dipentylguanidine (13.4 g.), 2-propanol (600 ml.) and water (150 ml.) was heated under reflux for 72 hours, then evaporated to dryness under reduced pressure. The residue was stirred with methanol (200 ml.), the undissolved residue of piperazine dihydrochloride was filtered off, and the filtrate was evaporated to dryness under reduced pressure. The residue was stirred with methanol (150 ml.), cyclohexane (150 ml.) and anhydrous sodium sulphate for 30 minutes at room temperature, the solid material was removed by filtration, and the filtrate was evaporated to

dryness, to give piperazine-1-[N-(N,N-dipentylamidino)-carboxamidine] dihydrochloride, which was used in the next process stage without further purification. (On t.l.c. on silica, $R_F$=0.6 in butanol: water: acetic acid, 12:5:3).

A mixture of piperazine-1-[N-(N,N-dipentyl-amidino)carboxamidine] dihydrochloride (3.92 g.), 3-cyano 1,1-di-isopentylguanidine (3.22 g.) and butanol (10 ml.) was heated under reflux for 18 hours. Acetone (200 ml.) was added, and the mixture was cooled to room temperature and stirred for 30 minutes. The solid was filtered off, washed with acetone (20 ml.) and dried in air at room temperature to give 4-[N-(N,N-dipentylamidino)-amidino]piperazine-1-[N-(N,N-diisopentylamidino)-carboxamidine] dihydrochloride, m.p. 236-237°.

Example 7

A two-phase mixture of piperazine dihydrochloride monohydrate (3.3 g.), 3-cyano-1-isobutyl-1-tetradecylguanidine (3.3 g.), butanol (120 ml.) and water (10 ml.) was stirred and heated under reflux for 14 hours. The mixture was cooled, acetone (250 ml.) was added, and the mixture was heated under reflux for a further 30 minutes, then cooled. The solid product thus obtained was filtered off and dissolved in butanol (100 ml.) and the solution was washed with 2N aqueous hydrochloric acid (5 ml.), then concentrated to a small volume. Acetone (90 ml.) was added to precipitate N-(N-isobutyl-N-tetradecylamidino)piperazine-1-carboxamidine dihydrochloride dihydrate, m.p. 180-185°.

N-(N-Isobutyl-N-tetradecylamidino)piperazine-1-carboxamidine dihydrochloride dihydrate (1.52 g.) and 3-cyano-1,1-dimethylguanidine (0.41 g.) in butanol (10 ml.) were stirred and heated under reflux for 5 hours. A further quantity of the cyanoguanidine (0.04 g.) was added and the mixture was stirred for a further 2 hours. Butanol (10 ml.) was added, the mixture was filtered hot, and on cooling the filtrate gave 4-[N-(N,N-dimethyl-

amidino)amidino]-N-(N-isobutyl-N-tetradecylamidino)-piperazine-1- carboxamidine dihydrochloride, m.p. 233.5-235.5°.

The 3-cyano-1-isobutyl-1-tetradecylguanidine used as starting material in the above process was manufactured from N-isobutyltetradecylamine and sodium dicyanamide, by the process described generally in the second part of Example 1, and was obtained as a waxy solid, m.p. 54-55°, from toluene.

Example 18

A mixture of 3-cyano-1-isobutyl-1-tetradecyl-guanidine (3.36 g.), N-cyanomorpholine-1-carboxamidine (1.54 g.), piperazine dihydrochloride monohydrate (1.77 g.) and propanol (50 ml.) was stirred and heated under reflux for 20 hours, then filtered and evaporated to dryness. The residual gum was purified by reverse phase MPLC on silica (ODS $C_{18}$ grade) on a column 30 cm. x 3 cm, eluting with methanol: water, 55:45 by volume, at 25 ml./minute. The fractions collected between 50 minutes and 70 minutes from commencement were combined and evaporated to dryness, and the residue was crystallised from a mixture of equal volumes of methanol and water to give N-(N-isobutyl-N-tetradecylamidino)-4-[N-morpholino-carbonimidoyl)amidino]piperazine-1-carboxamidine dihydrochloride, m.p. 231.5-234°.

Example 19

A mixture of 3-cyano-1-dodecyl-1-methylguanidine (1.99 g.), 3-cyano-1,1-dimethylguanidine (0.84 g.) and piperazine dihydrochloride monohydrate (1.32 g.) was heated under reflux in butanol for 3 hours, and the major component was isolated by reverse phase MPLC, using the procedures described generally in Example 19, to give N-(N-dodecyl-N-methylamidino)-4-[N-(N,N-dimethyl-amidino)amidino]piperazine-1-carboxamidine dihydrochloride, m.p. 235-236.5°.

The 3-cyano-1-dodecyl-1-methylguanidine used as starting material in the above process was manufactured

from N-methyldodecylamine and sodium dicyanamide by the process described generally in the second part of Example 1, and was obtained from petroleum ether (b.p. 60-80°.) solution as a solid, m.p. 76-88°.

Example 20

A solution of piperazine dihydrochloride monohydrate (0.6 g.) and 3-cyano-2-hexyl-1,1-dimethylguanidine (1.65 g.) in propanol (25 ml.) was heated under reflux for 18 hours, then cooled and evaporaed to dryness. The residue was triturated with acetonitrile and the solid obtained was filtered off and dried to give piperazine-1,4-di[N-(N-hexyl-N',N'-dimethylamidino)-carboxamidine] as a white solid m.p. 237° (decomposition).

The 3-cyano-2-hexyl-1,1-dimethylguanidine used as starting material in the above process may be prepared as follows:-

N-cyano-S,S'-dimethyl-iminodithiocarbonate (2 g.) in ethanol (20 ml.) was added hexylamine (1.8 ml.). The mixture was stirred at room temperature for 2 hours and then evaporated to dryness. The residue was dissolved in a solution of dimethylamine in ethanol (33% w/v -20ml.), and the mixture was allowed to stand at room temperature for 18 hours and then evaporated to dryness, to give 3-cyano-2-hexyl-1,1-dimethylguanidine as a brown oil which was used without further purification.

Examples 21-25

The process described in Example 20 was repeated, using the appropriate cyanoguanidine starting materials, to give the following dihydrochlorides of compounds of the formula I ($R^{15}=R^{16}=H$, m=2):-

| Ex.No. | $R^{10}(-R^{12})$ | $R^{11}(=R^{13})$ | $R^{14}(=R^{17})$ | m.p.(°) |
|---|---|---|---|---|
| 21 | $-(CH_2)_5-$ | | hexyl | 259-261 |
| 22 | $-(CH_2)_6-$ | | pentyl | 277-279 |
| 23 | i-pentyl | i-pentyl | methyl | 260-262 |
| 24 | butyl | butyl | methyl | 273-276 |
| 25 | pentyl | pentyl | methyl | 250-252 |

The cyanoguanidine starting materials used in the manufcture of the above compounds were prepared by reacting N-cyano-S,S'-dimethyl-iminodithiocarbonate successively with the required amines, by the process decribed generally in the second part of Example 20.

Example 26

A mixture of piperazine dihydrochloride (1.3 g.) and 2,3,4,5,6,7-hexahydro-1,3-di-isopentyl-1H-1,3-diazepin-2-ylimine carbonitrile (4.9 g.) were fused together at 150° for 1 hour. Butanol (250 ml.) was added and the mixture was stirred and heated under reflux for 72 hours. The solvent was evaporated, and the residue was purified by reverse phase MPLC, using methanol: water, 55:45 by volume, as the eluant. The fractions containing the product having the lowest $R_F$ (0.5) on thin layer chromatography on silica, eluting with a mixture of butanol (6 parts), acetic acid (1.5 parts) and water (2.5 parts) were combined and evaporated to dryness to give 1,4-bis[N-(2,3,4,5,6,7-hexahydro-1,3-di-isopentyl-1H-1,3-diazepin-2-ylidene)amidino]piperazine dihydrochloride, m.p. 276-277°.

The 2,3,4,5,6,7-hexahydro-1,3-di-isopentyl-1H-1,3-diazepin-2-ylimine carbonitrile, used as starting material in the above process, may be made as follows:-

Isopentylamine (100 ml. of a 30% solution in water) was added dropwise to stirred N-cyano-5,5'-dimethyl-iminodithiocarbonate (10 g.) at room temperature. The mixture was heated under reflux for 16 hours, the solvent was evaporated under reduced pressure, and the residue was crystallised from petroleum ether, (b.p. 60-80°), to give 1-cyano-2,3-di-isopentylguanidine, m.p. 75-76°.

1-Cyano-2,3-di-isopentylguanidine (5.0 g.) was dissolved in dimethylformamide (50 ml.) under an atmosphere of argon, and sodium hydride (2.14 g.) of a 50% dispersion in oil) was added in portions. After 1 hour, when effervescence had subsided, 1,4-dibromobutane (4.8 g.) was added dropwise, and the reaction mixture was stirred for 16 hours. Water (100 ml.) was added, and the mixture was extracted with ethyl acetate (3 x 50 ml.). The extracts were combined, washed with water (3 x 50 ml.), dried and evaporated to dryness under reduced pressure to give the required carbonitrile starting material, as an oil.

Example 27

A solution of piperazine dihydrochloride monohydrate (0.84 g.) and 1-butyl-3-cyano-2-hexyl-1,2-dimethylguanidine (3.4 g.) in propanol (8 ml.) was heated under reflux for 6 days. The mixture was cooled, added to water (20 ml.), and extracted with chloroform (20 ml.). The chloroform extract was evaporated to dryness and the residue was triturated with ethyl acetate to give piperazine-1,4-di[N-(N-butyl-N'-hexyl-N,N'-dimethylamidino)carboxamidine]dihydrochloride as a white solid, m.p. 194-197°.

The 1-butyl-3-cyano-2-hexyl-1,2-dimethylguanidine, used as starting material in the above process may be prepared as follows:-

To a solution of N-cyano-S,S'-dimethyl-iminodithiocarbonate (4.8 g.) in ethanol (25 ml.) was added N-methylhexylamine (3.8 g.). The mixture was allowed to

stand for 18 hours, and was then evaporated to dryness. The residue was dissolved in a solution of methylamine in ethanol (33% w/v - 100 ml.) and allowed to stand for 3 hours. The mixture was then evaporated to dryness and the residue (4.0 g.) was dissolved in dimethylformamide (20 ml.). To this solution was added sodium hydride (50% dispersion in oil - 1.02 g.) in portions. After the evolution of hydrogen had ceased, the mixture was treated with butyl bromide (2.74 g.). The mixture was then allowed to stand for 18 hours and added to water (50 ml.). The aqueous mixture was extracted with ethyl acetate (2 x 50 ml.) and the ethyl acetate extracts were combined, washed with water (2 x 50 ml.) and dried over magnesium sulphate, and the solution was evaporated to dryness. The residue was purified by chromatography on silica, using mixture of ethyl acetate/petrol ether (1:1 by volume) as eluant. The appropriate fractions were evaporated to dryness to give 1-butyl-3-cyano-2-hexyl--1,2-dimethylguanidine as a clear oil.

<u>Examples 28-32</u>

The process described in Example 27 was repeated, using the appropriate cyanoguanidines as starting materials, to give the following compounds:-

| Ex.No. | $R^{10}$ (-$R^{12}$) | $R^{11}$ (=$R^{13}$) | $R^{14}$ (=$R^{16}$) | $R^{15}$ (=$R^{17}$) | Reaction time (days) | m.p.° |
|---|---|---|---|---|---|---|
| 28 | hexyl | methyl | propyl | methyl | 4 | (a) |
| 29 | heptyl | propyl | methyl | methyl | 7 | 220-222 |
| 30 | pentyl | pentyl | methyl | methyl | 7 | 216-218 |
| 31 | decyl | methyl | $-(CH_2)_5-$ | | 6 | (b) |
| 32 | heptyl | methyl | methyl | methyl | 7 | (c) |

(a) Mass ion = 562, (calculated-562) by fast atom bombardment mass spectrometry.

(b) Purified by reverse phase MPLC to give a tetra-acetate. NMR in deuteriochloroform ( $\delta$ values):- 0.8, triplet, 6H; 1.2, singlet, 32H; 1.6, singlet, 12H; 2.0, singlet, 12H; 2.9, singlet, 6H; 3.2, broad singlet, 12H; 3.7, singlet, 8H.

(c) Purified by reverse phase MPLC to give a tetra-acetate. NMR in deuteriochloroform ( $\delta$ values):- 0.8, triplet, 6H; 1.2, singlet, 16H; 1.5, broad singlet, 4H; 1.95, singlet, 12H; 2.8, singlet, 18H; 3.1, multiplet, 4H; 3.7, singlet, 8H.

Example 33

The process described in Example 1 was repeated, using piperazine dihyrochloride in place of the dimesylate to give piperazine-1,4-di[N-(N,N-dipentylamidino)carboxamidine] dihydrochloride, m.p. 239-240°, crystallised from water.

Example 34

Piperazine-1,4-di[N-(N,N-dipentylamidino)-carboxamidine] dihydrochloride (22.7 g.) was dissolved in isopropyl alcohol (33 ml.) and water (18 ml.) at 55°. Aqueous 2N sodium hydroxide solution (41.7 ml.) was added, and the mixture was stirred and warmed until a clear solution was obtained, then allowed to cool slowly to 40°. The solution was then placed in a water bath at 25° and the product was allowed to crystallise slowly. The bath temperature was lowered to 15° and the crystalline product was filtered off, washed with water (2 x 23 ml.) and air dried to give piperazine-1,4-di[N-(N,N-dipentylamidino)carboxamidine] as the free base, m.p. 85-90°C.

Example 35

Piperazine-1,4-di[N-(N,N-dipentylamidino)-carboxamidine] dihydrochloride (10 g.) was mixed with deionised water (100 ml.) on a steam bath until dissolved. A solution of potassium bicarbonate (132 g.) in deionised water (1,250 ml.) was heated to 65° and added quickly to give a clear solution. The resulting solution was stirred and cooled rapidly in an ice water bath to form a precipitate which was filtered and washed with water to give, after drying under reduced pressure at 35°, piperazine-1,4-di[N-(N,N-dipentylamidino)-carboxamidine] bis(bicarbonate), m.p. 117-120°.

Example 36-41

A solution of piperazine-1,4-di[N-(N,N-di-pentylamidino)carboxamidine] bis(bicarbonate) (2.5 g.) in water (100 ml.) was added to a suspension of sulphadiazine (1.75 g.) in water (100 ml.) The mixture was boiled and stirred for 10 minutes. On cooling, an oil separated which crystallised on standing to give the bis(sulphadiazine) salt, m.p. 91°, crystallised from aqueous methanol (Example 36).

In a similar manner, there were obtained the following salts of the compound described in Example 1:-

| Ex. | Salt | Crystallistion Solvent | m.p.(°) |
|---|---|---|---|
| 37 | di-octanoate | methyl isobutyl ketone | 145-146 |
| 38 | dipalmitate | methyl isobutyl ketone | 87-88 |
| 39 | digluconate | (a) | 80-86 |
| 40 | dipivalate | (amorphous) | 165-16 |
| 41 | tartrate | water | 135-141 |

(a) - freeze-dried from an aqueous solution; hygroscopic.

Examples 42-46

The following salts of the compound described in Example 1 were prepared by reacting the free base, obtained as described in Example 34, with the appropriate acid:-

| Ex. | Salt | Crystallisation Solvent | m.p.(°) |
|---|---|---|---|
| 42 | bis(ethanesulphonate) | water | 225-226 |
| 43 | bis(pyroglutamate) | (amorphous) | 160-166 |
| 44 | diacetate | acetone/isopropyl alcohol | 188-189 |
| 45 | glutamate | (amorphous) | 115-117 |
| 46 | disulphamate | water | 200-201 |

Example 47

The free base prepared in Example 34 (44 g. of solid containing 9.2% water) was added to a mixture of 4-methylpentan-2-one (380 ml.) and D,L-lactic acid (15.3g.) at 88% w/w strength). A clear solution was formed, with pH about 4. Water present in the mixture was removed by azeotropic distillation using a binary separator; the temperature of the mixture was allowed to rise to 115° with the product starting to crystallise out at 107°. The mixture was allowed to cool slowly to room temperature and then stirred for 30 minutes at 10°. The white solid was filtered off, washed with cold 4-methylpentan-2-one (2 x 15 ml.) and then allowed to dry in air at room temperture, to give the dilactate salt of the compound of Example 1, m.p. 207-207.5°.

What we claim is:

1. A diaz(ep)ine bis(N-amidinoamidine) derivative of the formula:-

$R^{10}R^{11}N.C(NR^{14}R^{15}):N.C(:NH)-$ 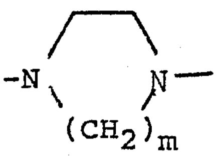

$C(:NH)N:C(NR^{16}R^{17})-NR^{12}R^{13}$

or a tautomeric form thereof, wherein $R^{10}$, $R^{11}$, $R^{12}$, $R^{13}$, $R^{14}$, $R^{15}$, $R^{16}$ and $R^{17}$, which may be the same or different, are each hydrogen, a 1-16C alkyl radical, a 3-18C cycloalkyl or cycloalkylalkyl radical, or an optionally-substituted phenyl (1-4C alkyl) radical, or $R^{10}$, $R^{11}$ and the nitrogen atom to which they are attached, or $R^{12}$ and $R^{13}$ and the nitrogen atom to which they are attached, or $R^{14}$ and $R^{15}$ and the nitrogen atom to which they are attached, or $R^{16}$ and $R^{17}$ and the nitrogen atom to which they are attached, which may be the same or different, are each a 1-azetidinyl, 1-pyrrolidinyl, piperidino, hexamethyleneimino, heptamethyleneimino, morpholino or 4-(1-8C alkanoyl)-1-piperazinyl radical, or $R^{10}$ and $R^{14}$ together with the two nitrogen atoms to which they are attached and the intervening carbon atom, or $R^{12}$ and $R^{16}$ together with the two nitrogen atoms to which they are attached and the intervening carbon atom, each forms a 1,3-diazaheterocyclic radical of 5 to 7 atoms, which heterocyclic radicals may be the same or different; and m is 2 or 3; provided that $R^{10}$, $R^{11}$, $R^{12}$, $R^{13}$, $R^{14}$, $R^{15}$, $R^{16}$ and $R^{17}$ together contain 16 to 28 carbon atoms, and the acid addition salts thereof.

2. A compound as claimed in claim 1 wherein each of $R^{10}$, $R^{11}$, $R^{12}$, $R^{13}$, $R^{14}$, $R^{15}$, $R^{16}$ and $R^{17}$, which may be the same or different, is hydrogen or a methyl, ethyl, propyl, isopropyl, butyl, isobutyl, s-butyl, test-butyl, pentyl, neopentyl, hexyl, heptyl, octyl, 2-ethylhexyl, decyl, dodecyl, tetradecyl, hexadecyl, cyclopentyl, cyclohexyl, cyclo-octyl, cyclododecyl, cyclohexadecyl, cyclopentylmethyl, cyclohexylmethyl, cyclo-octylmethyl, benzyl, phenethyl, 1-phenylethyl, 3-phenylpropyl, 1-phenylpropyl or 1-methyl-1-phenylethyl radical, or a benzyl, phenethyl, 1-phenylethyl, 3-phenylpropyl, 1-phenylpropyl or 1-methyl-1-phenylpropyl radical optionally substituted by chlorine, bromine, iodine or fluorine atoms or by amino, carbamoyl, cyano, hydroxy, nitro, trifluoromethyl, methyl, ethyl, propyl, butyl, pentyl, hexyl, methoxy, ethoxy, propoxy, acetamido, propionamido, butyramido, methylamino, ethylamino, propylamino, acetyl, propionyl, butyryl, methoxycarbonyl, ethoxycarbonyl, dimethylamino or diethylamino radicals.

3. A compound as claimed in claim 2 wherein any of $R^{10}$, $R^{11}$, $R^{12}$, $R^{13}$, $R^{14}$, $R^{15}$, $R^{16}$ and $R^{17}$, which may be the same or different, is a 2-, 3- or 4-chlorobenzyl, 2-, 3- or 4-bromobenzyl, 2-, 3- or 4-fluorobenzyl, 2,3-, 2,4-, 2,5-, 2,6-, 3,4- or 3,5-dichlorobenzyl, 2-chloro-4-fluorobenzyl, 2-, 3- or 4-methylbenzyl, 2,3-, 2,4-, 2,5-, 2,6-, 3,4- or 3,5-dimethylbenzyl, 2-, 3- or 4-methoxybenzyl, 2-, 3- or 4-acetylbenzyl, 2-, 3- or 4-methylaminobenzyl, 2-, 3- or 4-acetamido-benzyl, 2-, 3- or 4-methoxycarbonyl- benzyl, 2-, 3- or 4-dimethylaminobenzyl, 2-, 3- or 4-nitrobenzyl, 2-, 3 or 4-chloro-$\alpha$-methylbenzyl, 2-, 3- or 4-chlorophenethyl or 1-(4-chlorophenyl)-1-methylethyl radical.

4. A compound as claimed in claim 1 wherein m is 2.

5. A compound as claimed in claim 1 which is in the form of a hydrochloride, hydrobromide, phosphate, sulphate, acetate, D-gluconate, 2-pyrrolidone--5-carboxylate, mesylate, carbonate, bicarbonate, lactate, glutamate, octanoate, palmitate, pivalate, tartrate, ethanesulphonate, pyroglutamate, sulphamate or sulphadiazine salt.

6. A compound as claimed in claim 1 which is piperazine-1,4-di[N-(N,N-dipentylamidino)carboxamidine], piperazine-1,4-di[N-(N,N-di-isopentylamidino)carboxamidine], piperazine-1,4-di[N-(N-butyl-N-pentylamindino)carboxamidine], 4-[N-(N,N-dipentylamidino)amidino]piperazine-1-[N-(N,N-di-isopentylamidino)carboxamidine], piperazine-1,4-di(N-[N-butyl-N-(1,4-dimethylpentyl)amidino]carboxamidine), piperazine-1,4-di[N-($N^1N^1$-di-isopentyl-$N^2$-methylamidino)carboxamidine] or 1,4-bis[N-(2,3,4,5,6,7-hexahydro-1,3-di-isopentyl-1<u>H</u>- 1,3-diazepin-2-ylidene)-amidino]piperazine, or a dihydrochloride, dimesylate or dilactate thereof.

7. A process for the manufacture of a compound as claimed in claim 1 which comprises:-

a) for those compounds wherein $R^{14}$, $R^{15}$, $R^{16}$ and $R^{17}$ are all hydrogen, reacting piperazine-1,4-di(N-cyanoamidine or 2,3,4,5,6,7-hexahydro-1<u>H</u>-1,4-diazepine-1,4-di(N-cyanoguanidine) either with an amine $R^{10}R^{11}NH$, if the required product is symmetrical, or with an amine $R^{10}R^{11}NH$ and an amine $R^{12}R^{13}NH$, either simultaneously or sequentially, if the required product is unsymmetrical, the amine or amines being in the form of an acid addition salt thereof, wherein $R^{10}$, $R^{11}$, $R^{12}$ and $R^{13}$ have the meanings stated in claim 1, at a temperature of 100 to 170°; or

b) reacting piperazine or 2,3,4,5,6,7-hexahydro-

1*H*-1,4-diazepine in the form of an acid addition salt thereof either with a cyanoguanidine of the formula $R^{10}R^{11}N.C(NR^{14}R^{15}):N.CN$ if the required product is symmetrical, or with a cyanoguanidine $R^{10}R^{11}N.C(NR^{14}R^{15}):N.CN$ and a cyanoguanidine $R^{12}R^{13}N.C(NR^{16}R^{17}):N.CN$, either simultaneously or sequentially, if the required product is unsymmetrical, wherein $R^{10}$, $R^{11}$, $R^{12}$, $R^{13}$, $R^{14}$, $R^{15}$, $R^{16}$ and $R^{17}$ have the meanings stated in claim 1, at a temperature of 100 to 170°.

8. An antibacterial or antifungal composition comprising a compound as claimed in claim 1 and an inert diluent or carrier therefor.

9. A method of obtaining an antibacterial or antifungal effect:

(a) in medical and veterinary practice for the disinfection of wounds, membranes or skin tissue;

(b) in the sterilisation of surgical instruments and other medical apparatus and equipment;

(c) in toothpastes and mouthwashes for inhibiting gingivitis and the formation of dental plaque;

(d) in the disinfection of hard surfaces;

(e) in the disinfection of textiles;

(f) in the control of microbiological slime in the pulp and paper industries;

(g) in the control of micro-organisms in swimming pools, cooling water, pasteuriser water, aqueous oil emulsions and other circulating water systems; or

(h) against plant bacteria and/or fungi;

which comprises applying an antibacterially-effective amount of a bisbiguanide as claimed in claim 1 to the bacterially or fungally affected locus.

10. A contraceptive method which comprises applying to sperm, or the locus of sperm, a spermicidal, sperm-immobilising or mucospissic amount of a compound as claimed in claim 1.

DS32717
SC06
JDA/MJW: 9 Apr 84

European Patent Office

# EUROPEAN SEARCH REPORT

0125093
Application number

EP 84 30 2929

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | JOURNAL OF MEDICINAL CHEMISTRY, vol. 11, 1968, page 942, Washington, US; J.W. JAMES et al.: "The synthesis of some heterocyclic derivatives of biguanide with antibacterial activity"<br>* Page 942; page 943, table 1; page 944, table IV; page 945 *<br>--- | 1,2,7-10 | C 07 D 295/20<br>C 07 D 243/04<br>A 61 K 31/50<br>A 61 K 31/55 |
| A | DE-A-1 795 443 (ASPRO-NICHOLAS)<br>* Pages 1-10 *<br>----- | 1,2,7-10 | |
| | | | TECHNICAL FIELDS SEARCHED (Int. Cl. ³) |
| | | | C 07 D 295/20<br>C 07 D 243/00 |

The present search report has been drawn up for all claims

| Place of search | Date of completion of the search | Examiner |
|---|---|---|
| THE HAGUE | 14-08-1984 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82